(19) Europäisches Patentamt
European Patent Office
Office européen des brevets

(11) Publication number: 0 434 026 A1

(12) **EUROPEAN PATENT APPLICATION**

(21) Application number: **90124760.1**

(22) Date of filing: **19.12.90**

(51) Int. Cl.5: **G03C 1/83**, G03C 1/14, C09B 23/02, C07D 231/22, C07D 403/06

(30) Priority: **21.12.89 JP 332149/89**

(43) Date of publication of application: **26.06.91 Bulletin 91/26**

(84) Designated Contracting States: **BE DE FR GB IT NL**

(71) Applicant: **FUJI PHOTO FILM CO., LTD.**
**No. 210, Nakanuma Minami-Ashigara-shi**
**Kanagawa-ken(JP)**

(72) Inventor: **Adachi, Keiichi, c/o Fuji Photo Film Co., Ltd.**
**210, Nakanuma**
**Minami Ashigara-shi, Kanagawa(JP)**
Inventor: **Jimbo, Yoshihiro, c/o Fuji Photo Film Co., Ltd.**
**210, Nakanuma**
**Minami Ashigara-shi, Kanagawa(JP)**

(74) Representative: **Patentanwälte Grünecker, Kinkeldey, Stockmair & Partner**
**Maximilianstrasse 58**
**W-8000 München 22(DE)**

(54) **Methine compound.**

(57) A novel methine compound having a pyrazolone nucleus represented by the following general formula (I):

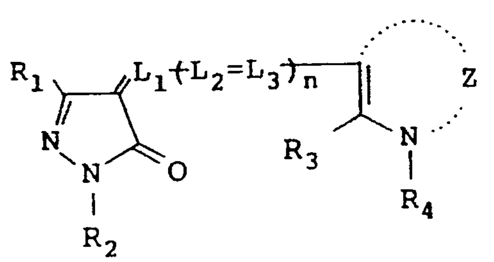

(I)

wherein $R_1$, $R_2$, $R_3$, $R_4$, Z, $L_1$, $L_2$, $L_3$ and n are defined in the specification.

EP 0 434 026 A1

# METHINE COMPOUND

## FIELD OF THE INVENTION

This invention relates to methine compounds having pyrazolone nuclei. More particularly, this invention relates to novel methine compounds which find use in such varied applications as dyestuffs, coloring matter for photographic use, coloring matter for use in filters, recording compounds for optical information recording media, staining agents for biological samples such as cells, pharmaceutical agents, and intermediates thereof.

## BACKGROUND OF THE INVENTION

Various methine compounds having pyrazolone nuclei have been disclosed in JP-A-56-12639 (the term "JP-A" as used herein means an "unexamined published Japanese patent application"), JP-A-54-118247, JP-A-63-197943, British Patent No. 584609, British Patent No. 695874, International Patent No. 88/04794, and other documents.

With regard to the application of these compounds in the field of photography, attempts have been made to add them to photographic materials, especially to silver halide photographic materials, to regulate spectral sensitivities or to prevent unsharp images caused by scattering of incident light (irradiation) or by reflection of light at the interface of a photographic emulsion and a support (halation). In many cases, however, these prior art compounds exert an undesirable influence upon the photographic emulsion or, in the case of multilayer photosensitive materials, they diffuse into adjacent layers and alter the photographic sensitivity. In addition, these compounds sometimes cause detrimental reactions due to their incomplete decomposition or their incomplete elution from the film materials during processing of the exposed films.

## SUMMARY OF THE INVENTION

The principal object of the present invention, therefore, is to provide a novel methine compound having a pyrazolone nucleus, which does not exhibit the undesirable characteristics of the known compounds.

This object is achieved by a compound represented by the following general formula (I):

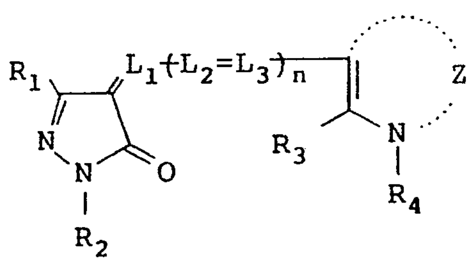

(I)

wherein $R_1$ is selected from the class consisting of an alkyl group, an aryl group, an alkoxy group, an alkoxycarbonyl group, an aryloxycarbonyl group, an acyl group, a hydroxy group, a cyano group, an acylamino group, a ureido group and an amino group; $R_2$ is an aryl group or a benzyl group; $R_3$ and $R_4$ represent independently a hydrogen atom, an alkyl group, an aryl group or an alkoxycarbonyl group; Z represents a non-metal atom which is necessary for the formation of a 5-membered heterocyclic ring, the heterocyclic ring optionally being made further into a benzo-condensed ring; n is 0 or 1; and $L_1$, $L_2$ and $L_3$ each represents a methine group; and wherein the compound contains at least one carboxyl group.

Preferably, the 5-membered heterocyclic ring formed via the non-metal atom Z in general formula (I) is selected from the group consisting of indole, pyrrole and pyrazole, and at least one carboxyl group is directly connected with an aryl group in the compound.

Other objects and advantages of the present invention will become apparent as the description progresses.

## DETAILED DESCRIPTION OF THE INVENTION

General formula (I) is described in detail below.

In general formula (I), $R_1$ is selected from the class consisting of an alkyl group having from 1 to 4 carbon atoms (for example, methyl, ethyl and t-butyl), an aryl group having from 6 to 7 carbon atoms (for example, phenyl, p-methylphenyl and p-carboxyphenyl), an alkoxy group having from 1 to 4 carbon atoms (for example, methoxy, ethoxy and n-butoxy), an alkoxycarbonyl group having from 2 to 5 carbon atoms (for example, methoxycarbonyl, ethoxycarbonyl and butoxycarbonyl), an aryloxycarbonyl group having from 7 to 8 carbon atoms (for example, phenoxycarbonyl and p-methylphenoxycarbonyl), an acyl group having from 2 to 5 carbon atoms (for example, acetyl and propionyl), a hydroxy group, a cyano group, an acylamino group having from 2 to 5 carbon atoms (for example, acetylamino and propionylamino), a ureido group having from 2 to 5 carbon atoms (for example, methylureido and ethylureido), and an amino group having from 0 to 6 carbon atoms (for example, amino, dimethylamino, methylamino and diethylamino).

$R_2$ is selected from the class consisting of an aryl group having from 6 to 10 carbon atoms (for example, phenyl, p-carboxyphenyl, 3,5-dicarboxyphenyl and p-carboxy-o-methoxyphenyl) and a benzyl group having from 7 to 10 carbon atoms (for example, benzyl, p-carboxybenzyl and p-chlorobenzyl).

$R_3$ and $R_4$ represent independently a hydrogen atom, an alkyl group having from 1 to 4 carbon atoms (for example, methyl, ethyl and methoxyethyl), an aryl group having from 6 to 12 carbon atoms (for example, phenyl, p-carboxyphenyl, p-dimethylaminophenyl and p-methoxyphenyl), or an alkoxycarbonyl group having from 2 to 4 carbon atoms (for example, methoxycarbonyl and ethoxycarbonyl).

Z represents a non-metal atom which is necessary for the formation of a 5-membered heterocyclic ring. The heterocyclic ring may optionally be made further into a benzo-condensed ring (for example, indole, pyrrole and pyrazole), and the heterocyclic ring may further have substituent groups. Finally, "n" in general formula (I) is 0 or 1, and $L_1$, $L_2$ and $L_3$ each represents a methine group. Each molecule represented by general formula (I) should contain at least one carboxyl group which, preferably, is connected directly with an aryl group in the molecule.

Preferably, general formula (I) is represented by the following general formula (II):

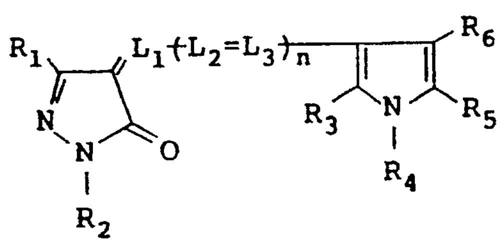

(II)

wherein $R_1$, $R_2$, $R_3$, $R_4$, $L_1$, $L_2$, $L_3$ and n have the same meaning as in general formula (I), respectively; and $R_5$ and $R_6$ represent independently a hydrogen atom, a substituted or unsubstituted alkyl group having from 1 to 4 carbon atoms (for example, methyl, ethyl and butyl), or a substituted or unsubstituted aryl group having from 6 to 10 carbon atoms (for example, phenyl, tolyl and chlorophenyl). $R_5$ and $R_6$ may optionally be linked with each other to form a benzo-condensed ring (for example, indole ring) together with the heterocyclic ring on which $R_5$ and $R_6$ are connected. Each molecule represented by general formula (II) should contain at least one carboxyl group.

Specific examples of the compounds of the present invention are illustrated below.

| | $R_1$ | $R_2$ | 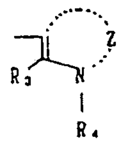 | n |
|---|---|---|---|---|
| 1 | $CH_3$ | 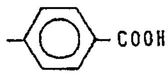 | 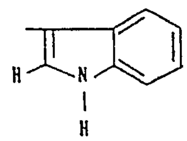 | 0 |
| 2 | $C_2H_5$ | 〃 | 〃 | 〃 |
| 3 | $C_2H_5O\overset{O}{\overset{\|}{C}}$ | 〃 | 〃 | 〃 |
| 4 | $C_2H_5O$ | 〃 | 〃 | 〃 |
| 5 | 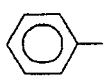 | 〃 | 〃 | 〃 |
| 6 | HO | 〃 | 〃 | 〃 |
| 7 | $CH_3$ | 〃 | 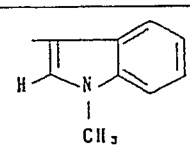 | 〃 |
| 8 | $C_2H_5$ | 〃 | 〃 | 〃 |
| 9 | $C_2H_5O\overset{O}{\overset{\|}{C}}$ | 〃 | 〃 | 〃 |

| | $R_1$ | $R_2$ | 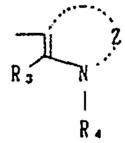 | n |
|---|---|---|---|---|
| 10 | $CH_3$ | 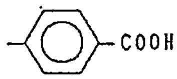 | 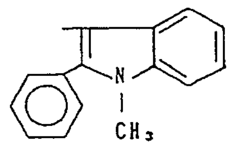 | 0 |
| 11 | $C_2H_5$ | 〃 | 〃 | 〃 |
| 12 | $CH_3$ | 〃 | 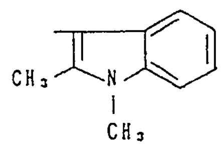 | 〃 |
| 13 | 〃 | 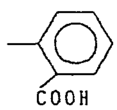 | 〃 | 〃 |
| 14 | 〃 | 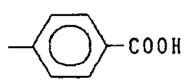 | 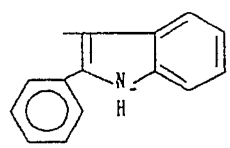 | 〃 |
| 15 | 〃 | 〃 | 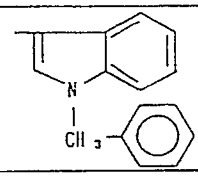 | 〃 |
| 16 | 〃 | 〃 | 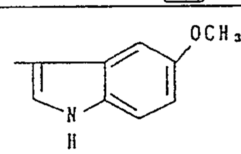 | 〃 |
| 17 | 〃 | 〃 | 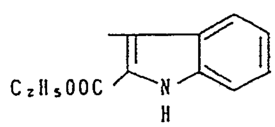 | 〃 |
| 18 | 〃 | 〃 | 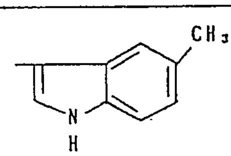 | 〃 |

| | $R_1$ | $R_2$ | 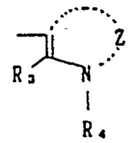 | n |
|---|---|---|---|---|
| 19 | 〃 | 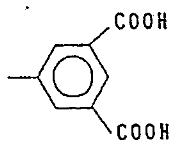 | 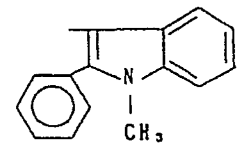 | 0 |
| 20 | 〃 | 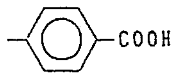 | 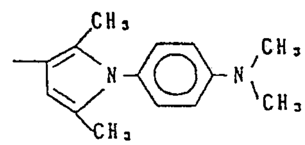 | 〃 |
| 21 | 〃 | 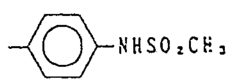 | 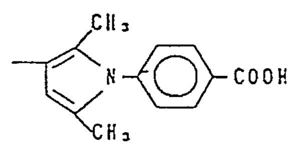 | 〃 |
| 22 | 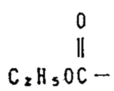 | 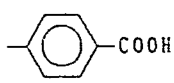 | 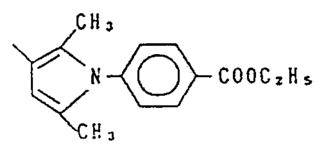 | 〃 |
| 23 | $CH_3$ | 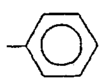 | 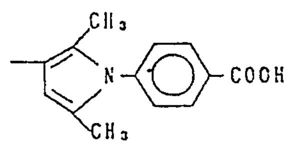 | 〃 |
| 24 | NC | 〃 | 〃 | 〃 |
| 25 | 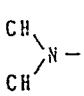 | 〃 | 〃 | 〃 |
| 26 | 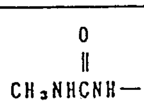 | 〃 | 〃 | 〃 |
| 27 | $CH_3$ | 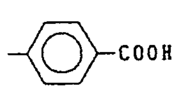 | 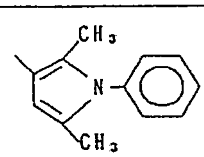 | 〃 |

| | $R_1$ | $R_2$ | 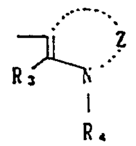 | n |
|---|---|---|---|---|
| 28 | 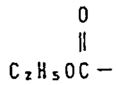 | 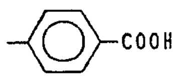 | 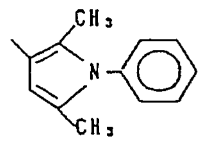 | 0 |
| 29 | $CH_3$ | 〃 | 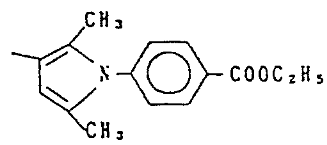 | 〃 |
| 30 | 〃 | 〃 | 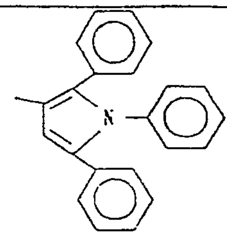 | 〃 |
| 31 | 〃 | 〃 | 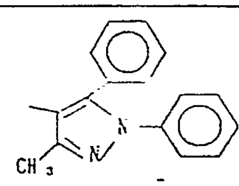 | 〃 |
| 32 | 〃 | 〃 | 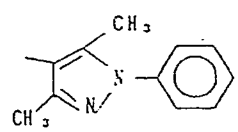 | 〃 |
| 33 | $CH_3CO-$ | 〃 | 〃 | 〃 |
| 34 | $CH_3$ | 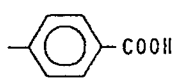 | 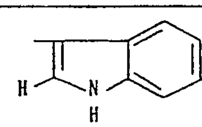 | 1 |
| 35 | 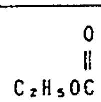 | 〃 | 〃 | 〃 |

| | $R_1$ | $R_2$ | 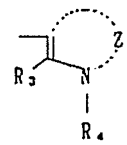 | n |
|---|---|---|---|---|
| 36 | $C_2H_5$ | 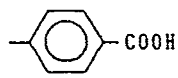 | 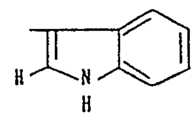 | 1 |
| 37 | $CH_3$ | 〃 | 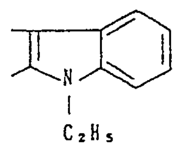 | 〃 |
| 38 | 〃 | 〃 | 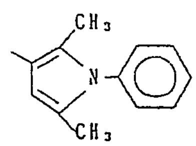 | 〃 |
| 39 | 〃 | 〃 | 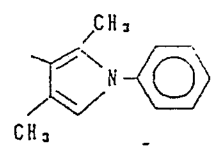 | 〃 |
| 40 | 〃 | 〃 | 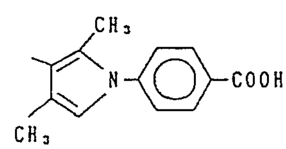 | 0 |

The compounds of the present invention may be obtained by means of a dehydration condensation of the following two compounds represented by general formulae (III) and (IV) by heating them in an organic solvent:

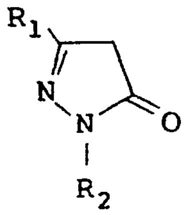

(III)

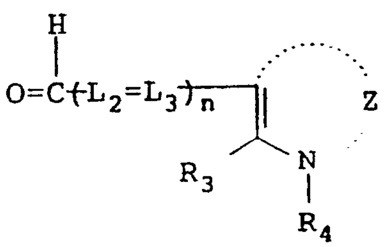

(IV)

wherein $R_1$, $R_2$, $R_3$, $R_4$, $L_2$, $L_3$ and Z have the same meaning as in general formula (I), respectively.

Preferred examples of the organic solvents include alcohols (for example, methanol, ethanol, propanol), esters (for example, ethyl acetate), acetonitrile, dimethylformamide (DMF), acetic acid, etc. The heating temperature can be selected from the range of from room temperature to a boiling temperature of solvent used.

When the reaction rate is slow, syntheses of the compounds of the present invention may be carried out in the presence of a small amount of acetic acid, acetic anhydride, triethylamine, ammonium acetate or the like as a catalyst.

The methine compounds of the present invention thus prepared are useful as dyestuffs, coloring matter for photographic use (especially as coloring matter for use in silver halide photosensitive materials (for use in filters, for the purposes of anti-irradiation and anti-halation, or the like)), recording compounds for optical information recording media, staining agents for biological samples such as cells, pharmaceutical agents, and intermediates thereof.

Examples of the present invention are given below by way of illustration and not by way of limitation.

## EXAMPLE 1

Five grams of 1-p-carboxyphenyl-3-methyl-5-pyrazolone (22.9 mmol) and 3.6 g of 3-formylindole (25.2 mmol) were mixed with 60 ml of ethanol and the mixture was subjected to reflux under heat for about 4 hours. After cooling the resulting reaction mixture to room temperature, the product was separated by filtration, washed with ethanol and then dried to obtain 7.3 g of the aforementioned illustrative compound 1 at a yield of 92.4%. Melting point: 300° C or above. DMF $\lambda$max: 450 nm ($\epsilon$, $2.70 \times 10^4$).

## EXAMPLES 2 TO 16

The compounds shown below were obtained in the same manner as Example 1.

| Compound | Melting point | DMF λmax (nm) |
|---|---|---|
| 2 | 300°C or above | 405 |
| 3 | 300°C or above | 468 |
| 4 | 300°C or above | 395 |
| 7 | 300°C or above | 410 |
| 10 | 299 to 300°C | 423 |
| 14 | 300°C or above | 442 |
| 20 | 300°C or above | 394 |
| 22 | 296 to 300°C | 441 |
| 23 | 257 to 258°C | 384 |
| 24 | 228 to 230°C | 452 |
| 27 | 300°C or above | 389 |
| 29 | 287 to 290°C | 386 |
| 31 | 298 to 299°C | 359 |
| 38 | 300°C or above | 445 |
| 40 | 300°C or above | 386 |

**EXAMPLE 17**

(Application to photographic light-sensitive material)

One gram of the illustrative compound 1 of the present invention obtained in Example 1 and 5 ml of a 5% aqueous solution of

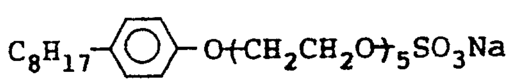

were kneaded and then made into fine particles using a sand mill. The thus prepared fine particles were dispersed in 25 ml of a 10% gelatin solution in which 0.5 g of citric acid was dissolved. The gelatin was treated with lime prior to its use. After removing sand using a glass filter, the fine particle dispersion was mixed with 10 ml of a 7% gelatin solution. At this stage, the mean particle size was found to be 0.15 μm.

Next, the thus prepared fine particle dispersion was mixed with a hardening agent (sodium salt of 2,4-dichloro-6-hydroxy-1,3,5-triazine) and the mixture was applied to a transparent polyethylene terephthalate support in such a way that the compound of the present invention was coated on the support at a density of 0.1 g/m$^2$. The thus prepared film sample was immersed for 30 seconds in an aqueous solution of pH 10, the same condition as the treating condition for the development of films, in order to find the degree of disappearance of color of the compound from the film sample. When absorption spectra of the compound taken before and after the immersion were compared, only trace amounts of residual compound were

detected in the film sample which had been immersed.

**EXAMPLE 18**

Film samples were prepared in the same manner as in Example 17. Each of the samples thus prepared was immersed for 20 seconds in an aqueous solution of pH 11 and then dried. Optical densities of the film samples before and after the immersion were measured by using a spectrophotometer for ultraviolet and visible region light, in order to calculate the degree of compound remaining after the immersion according to the following formula.

$$\text{Remaining degree A} = \frac{\text{Optical density after immersion}}{\text{Optical density before immersion}}$$

Each optical density was measured at the maximum absorption wavelength of the sample. Examples of the results are summarized below.

| Compound | A | Compound | A |
|---|---|---|---|
| 1 | 0.00 | 22 | 0.01 |
| 2 | 0.01 | 23 | 0.01 |
| 3 | 0.01 | 24 | 0.00 |
| 4 | 0.01 | 27 | 0.00 |
| 7 | 0.00 | 29 | 0.01 |
| 10 | 0.02 | 31 | 0.03 |
| 14 | 0.02 | 38 | 0.03 |
| 20 | 0.01 | 40 | 0.00 |

The above table shows that the amounts of the compound of the present invention remaining after the immersion were so small that they could hardly be detected when the film samples were treated under conditions similar to an actual film development process.

Thus, it is apparent that the present invention provides a novel methine compound having a pyrazolone nucleus, which is especially useful as coloring matter for photographic use, and which is readily removed when a photographic film coated with the compound is processed under normal conditions for development.

While the invention has been described in detail and with reference to specific embodiments thereof, it will be apparent to one skilled in the art that various changes and modifications can be made therein without departing from the spirit and scope thereof.

## Claims

1. A methine compound represented by the following general formula (I):

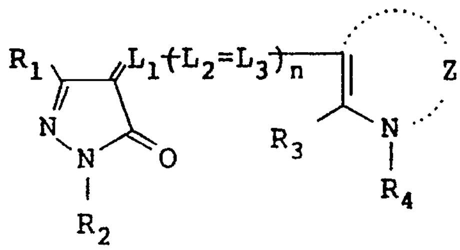

(I)

wherein $R_1$ is selected from the class consisting of an alkyl group, an aryl group, an alkoxy group, an alkoxycarbonyl group, an aryloxycarbonyl group, an acyl group, a hydroxy group, a cyano group, an acylamino group, a ureido group and an amino group; $R_2$ is an aryl group or a benzyl group; $R_3$ and $R_4$ represent independently a hydrogen atom, an alkyl group, an aryl group or an alkoxycarbonyl group; Z represents a non-metal atom which is necessary for the formation of a 5-membered heterocyclic ring, the heterocyclic ring optionally being made further into a benzo-condensed ring; n is 0 or 1; and $L_1$, $L_2$ and $L_3$ each represents a methine group; and wherein the compound contains at least one carboxyl group.

2. A methine compound represented by the general formula (I) according to claim 1 wherein $R_1$, $R_2$, $R_3$, $R_4$, $L_1$, $L_2$, $L_3$ and n have the same meaning as in claim 1, respectively; the 5-membered heterocyclic ring formed via the non-metal atom Z is selected from the group consisting of indole, pyrrole and pyrazole; and at least one carboxyl group is directly connected with an aryl group in the compound.

3. A methine compound according to claim 1, wherein general formula (I) is represented by the following general formula (II):

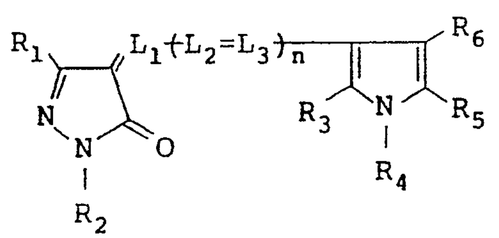

(II)

wherein $R_1$, $R_2$, $R_3$, $R_4$, $L_1$, $L_2$, $L_3$ and n have the same meaning as in claim 1, respectively; $R_5$ and $R_6$ represent independently a hydrogen atom, a substituted or unsubstituted alkyl group having from 1 to 4 carbon atoms, or a substituted or unsubstituted aryl group having from 6 to 10 carbon atoms; provided that $R_5$ and $R_6$ may optionally be linked with each other to form a benzo-condensed ring together with the heterocyclic ring on which $R_5$ and $R_6$ are connected; and provided that each molecule contain at least one carboxyl group.

European
Patent Office

# EUROPEAN SEARCH REPORT

Application Number

**EP 90 12 4760**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,Y | US-A-3 627 532 (DEPOORTER ET AL.)<br>* the whole document *<br>– – – | 1-3 | G 03 C 1/83<br>G 03 C 1/14<br>C 09 B 23/02<br>C 07 D 231/22<br>C 07 D 403/06 |
| X,Y | US-A-3 441 563 (WEISSEL ET AL.)<br>* column 2, lines 22 - 44; claim 1 *<br>– – – | 1-3 | |
| Y | JP-A-5 596 941 (MITSUBISHI)<br>* abstract * * page 3, right-hand column *compounds (VII),(VIII) *<br>– – – – – | 1-3 | |

TECHNICAL FIELDS SEARCHED (Int. Cl.5)

G 03 C
C 09 B
C 07 D

The present search report has been drawn up for all claims

| Place of search | Date of completion of search | Examiner |
|---|---|---|
| The Hague | 07 March 91 | MAGRIZOS S. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document